(19) Europäisches Patentamt
European Patent Office
Office européen des brevets

(11) Veröffentlichungsnummer: **0 550 807 A2**

# (12) EUROPÄISCHE PATENTANMELDUNG

(21) Anmeldenummer: **92118499.0**

(51) Int. Cl.$^5$: **A61K 31/12**

(22) Anmeldetag: **29.10.92**

(30) Priorität: **14.11.91 DE 4137540**

(43) Veröffentlichungstag der Anmeldung:
**14.07.93 Patentblatt 93/28**

(84) Benannte Vertragsstaaten:
**AT BE CH DE DK ES FR GB GR IE IT LI LU NL PT SE**

(71) Anmelder: **STEIGERWALD ARZNEIMITTELWERK GMBH**
**Havel Strasse 5**
**W-6100 Darmstadt(DE)**

(72) Erfinder: **Ammon, H.P.T., Prof. Dr. med.**
**Im Kleeacker 30**
**W-7400 Tübingen(DE)**
Erfinder: **Safayhi, Hasan, Dr.**
**Eichenweg 5**
**W-7400 Tübingen(DE)**
Erfinder: **Okpanyi, Samuel Nwachukwu, Dr.**
**Karl-Josef-Schlitt-Strasse 2**
**W-6200 Wiesbaden(DE)**

(74) Vertreter: **Kraus, Walter, Dr. et al**
**Patentanwälte Kraus, Weisert & Partner**
**Thomas-Wimmer-Ring 15**
**W-8000 München 22 (DE)**

## (54) Verwendung von Präparaten der Curcuma-Pflanzen.

(57) Die Erfindung betrifft die Verwendung von Präparaten der Pflanze Curcuma longa oder von anderen Pflanzen, die Curcumin enthalten, Curcumin oder Curcuminderivaten zur Prophylaxe und/oder Bekämpfung von Krankheiten, die mit einer übermäßigen Bildung von Leukotrienen und/oder Prostaglandinen einhergehen, sowie die Verwendung von Präparaten der Pflanze Curcuma longa oder von anderen Pflanzen, die Curcumin enthalten, Curcumin oder Curcuminderivaten zur Herstellung von Arzneimitteln für die Prophylaxe und/oder Bekämpfung von Krankheiten, die mit einer übermäßigen Bildung von Leukotrienen und/oder Prostaglandinen einhergehen.

EP 0 550 807 A2

Die Erfindung betrifft die Verwendung von Präparaten der Pflanze Curcuma longa oder von anderen Pflanzen, die Curcumin enthalten, Curcumin oder Curcuminderivaten zur Prophylaxe und/oder Bekämpfung von Krankheiten, die mit einer übermäßigen Bildung von Leukotrienen und/oder Prostaglandinen einhergehen. Sie betrifft ebenfalls die Verwendung von Präparaten der Pflanze Curcuma longa oder von anderen Pflanzen, die Curcumin enthalten, Curcumin oder Curcuminderivaten zur Herstellung von Arzneimitteln für die Prophylaxe und/oder Bekämpfung von Krankheiten, die mit einer übermäßigen Bildung von Leukotrienen und/oder Prostaglandinen einhergehen.

Erfindungsgemäß erfolgt die Verwendung insbesondere bei chronisch entzündlichen Darmerkrankungen, wie Colitis ulcerosa oder Morbus Crohn, und bei chronischer Hepatitis, chronischem Asthma bronchiale oder Psoriasis.

Beispielsweise handelt es sich bei Morbus Crohn und bei der Colitis ulcerosa um zwei chronisch entzündliche Darmerkrankungen, die mit der bisher zur Verfügung stehenden Therapie nicht oder nur ungenügend beeinflußt werden können. Pathophysiologisch gesehen wird angenommen, daß eine übermäßige Bildung sogenannter Leukotriene (Entzündungsmediatoren, die insbesondere als Lockstoffe für weiße Blutzellen dienen) die Hauptursache dafür sind, daß diese Erkrankungen am Laufen gehalten werden. Bisher gibt es praktisch kein wirksames Medikament gegen entzündliche Darmkrankheiten. Zur Verfügung stehen zwar sogenannte Glucocorticoide (Derivate von Nebennierenrindenhormonen), insbesondere Cortison. Sie sind jedoch mit erheblichen Nebenwirkungen behaftet, so daß ihr Einsatz nicht unproblematisch ist. Auch die Anwendung von Sulfasalazin ist unbefriedigend. Sulfasalazin ist ein Stoff, der hauptsächlich die Bildung sogenannter Prostaglandine (ebenfalls Mediatoren der Entzündung) hemmt, die jedoch auf eine Weise in den Entzündungsvorgang eingreifen, der sich von dem der Leukotriene grundsätzlich unterscheidet. Durch Hemmung der Prostaglandin-Synthese kommt man bei diesen Erkrankungen nicht immer zum Ziel.

Chronisches Asthma und Psoriasis sind Krankheiten, für die bis heute keine befriedigend wirksamen Arzneimittel zur Verfügung stehen. Beide Krankheiten werden u.a. mit Cortison oder Cortisonderivaten behandelt. Die Verabreichung von Cortison und seinen Derivaten, insbesondere während längerer Zeit, ist jedoch mit erheblichen Nachteilen verbunden. Auch chronische Hepatitis kann nicht zufriedenstellend behandelt werden.

Es ist bekannt, daß Zellmembranbestandteile Phospholipide enthalten. Aus Phospholipiden der Zellmembranen wird durch Phospholipase A2 Arachidonsäure abgespalten, die ihrerseits durch Cyclooxygenase und Lipoxygenase in Prostaglandine und Leukotriene überführt wird. Die Prostaglandine und die Leukotriene halten die Entzündungen bei chronisch entzündlichen Darmerkrankungen, bei chronischer Hepatits, chronischem Asthma bronchiale oder Psoriasis am Laufen.

Der vorliegenden Anmeldung liegt die Aufgabe zugrunde, die Verwendung von Präparaten zur Verfügung zu stellen, die zur Prophylaxe und/oder Bekämpfung von Krankheiten, die mit einer übermäßigen Bildung von Leukotrienen und/oder Prostaglandinen einhergehen, dienen. Die erfindungsgemäß verwendeten Präparate sollen nur gering toxisch sein und von den Patienten gut toleriert werden. Sie sollen weiterhin auf einfache Weise verfügbar sein.

Die Anmelderin hat überraschenderweise gefunden, daß Curcumin oder seine Derivate oder Präparate von Pflanzen der Curcuma longa oder von anderen Pflanzen, die Curcumin enthalten, die entzündlichen Krankheiten, die durch Bildung von Leukotrienen und Prostaglandinen verursacht werden, hemmt.

Gegenstand der Erfindung ist somit die oben genannte Verwendung.

Die Anmelderin hat im Zusammenhang mit Untersuchungen zum Mechanismus der allgemein antiphlogistischen Wirkung, beschrieben am Tiermodell des Rattenpfotenödems (Übersicht bei Ammon u. Wahl, 1990), pharmakologische Versuche durchgeführt und die folgenden biochemischen Befunde erhalten:

Curcumin hemmt in vitro die Aktivität:

(a) des Schlüsselenzyms der Leukotrien-Biosynthese (5-Lipoxygenase) in stimulierten neutrophilen Granulocyten der Ratte mit einer halbmaximalen Hemmkonzentration ($IC_{50}$) von ca. 20 $\mu$mol/l. Die Figur 1 zeigt die Konzentrations-Wirkungs-Beziehung für die Hemmung der Produktion von Leukotrien $B_4$ aus endogenem Substrat in intakten neutrophilen Granulocyten der Ratte durch Curcumin [Methode: Safayhi, Tiegs, Wendel: Biochem. Pharmacol. 34:2691-2694, 1985];

(b) des Schlüsselenzyms der Prostanoid-Biosynthese (Prostaglandin/Thromboxan/Prostacyclin) in intakten, stimulierten humanen Blutplättchen mit einer $IC_{50}$ von ca. 2 $\mu$mol/l. Die Figur 2 zeigt die Konzentrations-Wirkungs-Beziehung für die Hemmung der Cyclooxygenase-Aktivität in intakten Blutplättchen durch Curcumin (Methode: Safayhi et al., JPET accepted);

(c) der 12-Lipoxygenase in intakten, stimulierten humanen Blutplättchen mit einer $IC_{50}$ von ca. 20 µmol/l. Die Figur 2 zeigt die Konzentrations-Wirkungs-Beziehung für die Hemmung der 12-Hydroxyeicosatetranoic acid (12-HETE)-Bildung in intakten Blutplättchen des Menschen durch Curcumin (Methode: Safayhi et al., JPET accepted);

(d) die Entstehung peroxidierter Arachidonsäure-Produkte im zellfreien System mit einer $IC_{50}$ von ca. 1 µmol/l. Die Figur 3 zeigt die Hemmung der Fe/Ascorbat-induzierten Entstehung oxidierter Produkte der Arachidonsäure durch Curcumin (Methode: Safayhi et al., JPET accepted).

Diese Ergebnisse verdeutlichen, daß Curcumin in intakten Zellen die Bildung von entzündungsfördernden Mediatoren aus Arachidonsäure (Leukotriene via 5-Lipoxygenase, 12-HETE via 12-Lipoxygenase und Prostaglandine via Cyclooxygenase) hemmt sowie die Entstehung toxischer Peroxide unterbindet. (a) bis (d) stellt die Kombination von entzündungshemmenden Mechanismen ein und derselben Substanz dar, für die es auf dem Arzneimittelmarkt nichts Vergleichbares gibt. Die Erkennung des Vorliegens dieser Kombination wird ebenfalls als Erfindung und für die Therapie der oben genannten Erkrankungen angesehen: Zwar dürfte die erhöhte Bildung von Leukotrienen bei den oben genannten Erkrankungen stark im Vordergrund stehen, so ist nicht zu übersehen, daß auch andere Entzündungsmediatoren, wie Prostaglandine und Sauerstoffradikale, entzündungsfördernd wirken (sie werden durch die antioxidativen Eigenschaften von Curcumin beseitigt). Die Tabelle zeigt eine Zusammenfassung wichtiger Entzündungsmediatoren.

**Tab. 1: Mediatoren und ihre Wirkungen. Die für pathophysiologische Vorgänge wichtigsten Wirkungen sind hervorgehoben.**

| | Gefäßtonus | Gefäß-permeabilität | Plättchen-aggregation | Leukozyten | Bronchien | Magen | Uterus | Schmerz |
|---|---|---|---|---|---|---|---|---|
| Histamin | **Dilatation*** Konstriktion* | **Zunahme** | | | **Konstriktion** | **Zunahme der Säureproduktion** | Kontraktion** Relaxation** | **Auslösung** |
| 5-Hydroxy-tryptamin | Dilatation* Konstriktion* | Zunahme** | Förderung | | Konstriktion** | | **Kontraktion*** | **Auslösung** |
| Bradykinin | **Dilatation** | **Zunahme** | | | **Konstriktion** | | **Kontraktion*** | **Auslösung** |
| C5a | **Kontraktion** | Zunahme | Förderung | **Chemotaxis** | Konstriktion | | | Auslösung |
| $PGE_2$ | Dilatation | **Zunahme** + | Hemmung° | | **Dilatation** | **Schutz** | | **Auslösung** + |
| $PGF_{2\alpha}$ | Kontraktion° | | | | Konstriktion | | **Kontraktion** | |
| $PGD_2$ | **Kontraktion** | | Hemmung | Chemotaxis | **Konstriktion** | | | **Auslösung** + |
| $PGI_2$ | **Dilatation** | Zunahme + | **Hemmung** | | | | | |
| $TXA_2$ | **Kontraktion** | | **Förderung** | | **Konstriktion** | | | |
| $LTB_4$ | | **Zunahme***** | | **Chemotaxis** | | | | |
| $LTC_4$ | **Kontraktion** | Zunahme + | | | **Konstriktion** | | | |
| $LTD_4$ | **Kontraktion** | Zunahme + | | | **Konstriktion** | | | |
| PAF | **Kontraktion** | **Zunahme** | **Förderung** | Chemotaxis | **Konstriktion** | Ulcerogene Wirkung | | |
| $\cdot O_2^-$ | Dilatation | **Zunahme***** | | **Chemotaxis** | | | | |

* In Abhängigkeit vom Kreislaufabschnitt und der Tierspezies
** In Abhängigkeit von der Tierspezies
\+ Bei Gegenwart anderer Mediatoren (z. B. Histamin, C5a)
*** Bei Gegenwart von neutrophilen Granulozyten
° Nur in hohen Konzentrationen

Die Skizze verdeutlicht die Angriffsorte der heute auf dem Markt verfügbaren Pharmaka, mit denen über die sogenannte Arachidonsäure-Kaskade in das Entzündungsgeschehen eingegriffen werden kann:

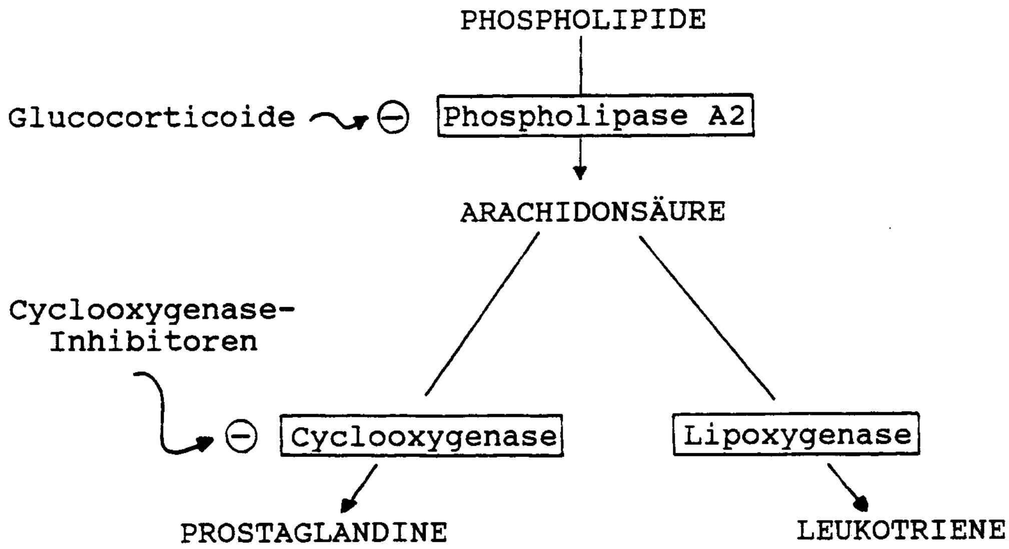


Glucocorticoide

Die Glucocorticoide hemmen zwar sowohl die Entstehung der Prostaglandine als auch der Leukotriene, verfügen jedoch als Abkömmlinge von Steroidhormonen, wie oben bereits erwähnt, über vielfältige gravierende Nebenwirkungen.

Nichtsteroidale Antiphlogistika

Nichtsteroidale Antiphlogistika (Acetylsalicylsäure, Indomethacin, Diclofenac, Ibuprofen usw.) hemmen durch Angriff im Bereich der Cyclooxygenase die Prostaglandin-Biosynthese. Sie können jedoch gerade hierdurch indirekt zur verstärkten Synthese von Leukotrienen führen und so Leukotrien-vermittelte pathophysiologische Reaktionen sogar verstärken (Beispiel: sog. Aspirin-Asthma). Beiden Inhibitor-Klassen (Glucocorticoide) fehlt die schützende Wirkung durch eine Peroxidentstehung-hemmende Komponente.

Überraschenderweise wurde gefunden, daß Curcumin und Curcumin enthaltende Präparate der Pflanze Curcuma longa oder von anderen Pflanzen, die Curcumin enthalten, oder Curcuminderivate sowohl die Prostaglandin als auch die Leukotrienentstehung hemmen, also im Wirkungsprofil hinsichtlich antiphlogistischer Eigenschaften mit den Steroiden vergleichbar sind, jedoch die unerwünschten Nebenwirkungen der Steroide und insbesondere von Cortison nicht zeigen. Dies wird im folgenden dargestellt:

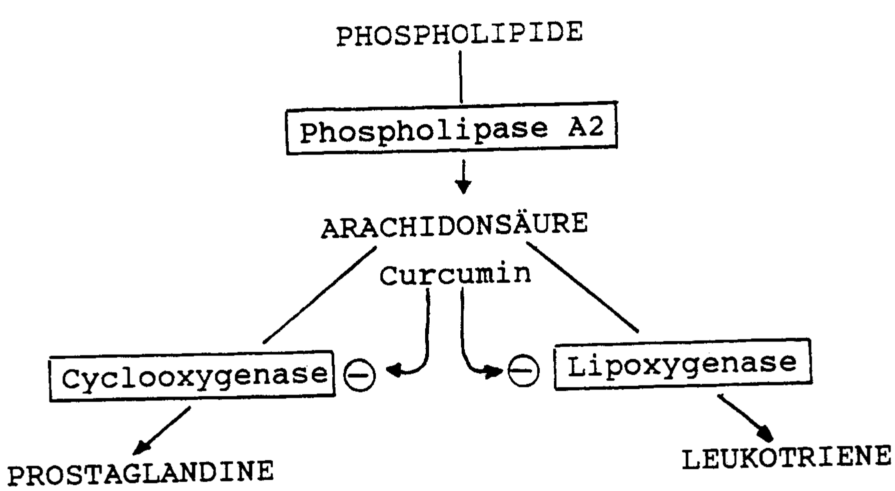

Die WirkungsweiSe von Curcumin ist jedoch grundsätzlich anders als bei den Steroidhormon-Abkömmlingen. Curcumin besitzt zusätzlich eine antioxidative Wirkungskomponente, von der eine weitere kurative Wirkung im Entzündungsgeschehen zu erwarten ist. Da Curcumin kein Hormon ist, sind auch die unerwünschten hormonbedingten Glucocorticoid-Nebenwirkungen nicht vorhanden, obwohl im Hinblick auf die entzündungshemmende Aktivität die erwünschten Steroidwirkungen erzielt werden.

Curcumin greift also in ein Entzündungsgeschehen in dreifacher Weise ein: Seine antioxidative Wirkung, die bereits mit einer Konzentration von 1 µm zu erzielen ist, dürfte sich wie folgt auswirken:

1. Die antioxidative Wirkung beseitigt aktivierte Sauerstoffmoleküle und Radikale. Für eine solche Wirkung gibt es bisher kein Medikament.
2. Durch die antioxidative Wirkung dürfte die Hemmung der Bildung von Cyclooxygenase-Produkten eintreten.
3. Durch die antioxidative Wirkung wird die Bildung von 5- und 12-Lipoxygenase-Produkten gehemmt.

Bei Curcumin handelt es sich daher um einen völlig neuen Typ eines Antiphlogistikums. Sein Vorteil ist, daß es neben den bei jeder Entzündung beteiligten aktivierten Sauerstoff-Species und den Cyclooxygenase-Produkten auch die Bildung von Leukotrienen hemmt.

Es war überraschend, daß Curcumin bei den genannten Krankheiten, insbesondere bei entzündlichen Darmkrankheiten, wirksam sein dürfte. In der Literatur wird beschrieben, daß Curcumin bei experimentellen Magengeschwüren der Ratte keine Besserung hervorrief. Bhatia et al. (Bhatia A., Singh G.B: und Khanna N.M. (1964), Indian J. Exptl. Biol. 2, 158-160) fanden keine Schutzwirkung von Curcumin bei Meerschweinchen bei Histamin-induzierten Magengeschwüren. Weiterhin haben Prasad et al. (Prasad, D.N. Gupta B., Srivastava, R.K. und Satyavati G.V. (1976), Indian J. Physiol. Pharmacol. 20, 92) eine Magenschleimhaut-schädigende Wirkung von hohen Dosen von Curcumin gefunden, wenn Curcumin in täglicher oraler Dosis von 100 mg/kg im Verlauf von 6 Tagen verabreicht wurde.

Prostaglandine besitzen in physiologischen Konzentrationen eine Magenschleimhaut-schützende Funktion. Prostaglandine üben auf die Darmschleimhaut (im Gegensatz zum Magen) keine Schutzfunktion aus. Die synchrone Hemmung der Entzündungs-Mediatoren (Prostaglandine und Leukotriene) sowie die Hemmung der Entstehung von Peroxiden durch Curcumin, insbesondere bei den Entzündungen der Darmschleimhaut (Colitis ulcerosa und Morbus Crohn), war daher in dieser Kombination für den Fachmann überraschend.

Erfindungsgemäß werden Präparate der Pflanze Curcuma longa, vorzugsweise Präparate, die aus Wurzeln oder Wurzelstöcken gewonnen werden, Präparate von anderen Pflanzen, die Curcumin enthalten, zum Beispiel Curcuma longa L. (Syn. Amomum curcuma Jacq., Curcuma domestica Lour., Curcuma xanthorriza Naves), Curcuma Xanthorrhiza Roxb. (Syn. Curcuma xanthorrhiza Dietrich, Curcuma xanthorrhiza Roxb.), natürliches oder synthetisches Curcumin oder Curcuminderivate verwendet. Beim Curcumin handelt es sich um den wesentlichen Inhaltsstoff der sogenannten Gelbwurz (Curcuma longa) sowie von Curcuma xanthorrhiza. Die Gelbwurz wird seit langem in den asiatischen Ländern insbesondere zur Behandlung von Dyspepsien (Verdauungsbeschwerden ohne nähere Charakterisierung) verwendet. Sie ist weiterhin Bestandteil des uns allen geläufigen Currygewürzes.

In folgenden Drogen sind Sesquiterpene enthalten, und in diesen kann auch Curcumin enthalten sein: Curcuma aromatica Salisb. (Syn. Curcuma zeodaria Roxb.), Curcuma Kwandsiensis Lee et Liang, Curcuma zedoaria (Berg.) Rosc. (Syn. Amomum zedoaria Berg., Amomum zerumbet Koen., Costus luteus Blanco, Curcuma nigricans Blanco, Curcuma zerumbet Roxb., Roscoea lutea Hassk., Roscoea nigrociliata Hassk.). Diese Substanzen können, sofern sie Curcumin enthalten, ebenfalls erfindungsgemäß verwendet werden.

Curcumin als solches wird darüber hinaus nach peroraler Gabe kaum im Blut gefunden. Curcumin eignet sich daher besonders zur lokalen Anwendung [Psoriasis, chronisches Asthma bronchiale (Spray, Inhalat)]. Da Curcumin in der Leber gefunden wird (Ammon und Wahl 1990), ist auch seine Anwendung bei chronisch entzündlicher Hepatitis möglich.

Bei Erkrankungen mit gesteigerter Leukotrien-Synthese, bei denen Curcumin nicht nach oraler Gabe wirksam ist, ist die parenterale Verabreichung oder die Anwendung resorbierbarer Derivate mit geringem "First Pass"-Effekt in Erwägung zu ziehen.

Curcumin hat die folgende Formel:

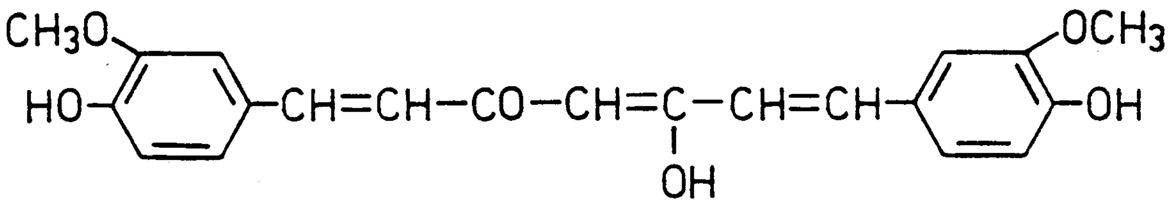

Curcumin zeigt praktisch keine toxische Wirkung bei einer Dosis von 5 g/kg bei oraler Verabreichung bei Meerschweinchen und Ratten.

Die erfindungsgemäßen Präparate können intraperitoneal, oral, rektal, intramuskulär, topisch, subkutan, intraartikulär oder intravenös verabreicht werden. Die orale Verabreichung ist bevorzugt. Die Verwendung kann in Form von Tabletten, Dragees, Kapseln, Lösungen, Emulsionen, Salben, Cremes, Inhalaten, Aerosolen oder Suppositorien erfolgen.

Erfindungsgemäß ist es weiterhin möglich, daß die Verwendung zusammen mit anderen chemisch reinen Arzneistoffen und/oder anderen pflanzlichen Arzneimitteln erfolgt.

Beispiele für solche andere chemisch reine Arzneistoffe sind:

BRONCHOLYTIKA UND ANTIASTHMATIKA

SYMPATHOMIMETIKA:

- Carbuterol-HCl
- Clenbuterol-HCl
- Fenoterol-HBr
- Isoetarin-HCl
- Orciprenalinsulfat
- Pirbuterol-HCl
- Procaterol-HCl
- Reproterol-HCl
- Sabutamolsulfat
- Terbutalinsulfat
- Tulobuterol-HCl

ANTIPSORIATIKA

NICHTSTEROIDALE ANTIPHLOGISTIKA:

- Salicylsäure und Derivate

VITAMINE:

- Folsäure
- Vitamin E
- Vitamin B12
- Vitamin A

VERSCHIEDENE:

- Cadmiumsulfid
- Benzalkoniumchlorid
- Natriumbituminosulfonat
- Ammoidin
- Allantoin
- Methotrexat

- Paraffin
- Tioxolon
- Dithranol
- Fumarsäure
- Undecylensäure
- Polyoxyethylenlaurylethersulfat
- Etretinat
- Zinkoxid
- Harnstoff
- Milchsäure

Die erfindungsgemäß verwendeten Zubereitungen können in an sich bekannter Weise unter Verwendung eines oder mehrerer pharmazeutisch annehmbarer Träger oder Verdünnungsmittel formuliert werden. Die Zubereitungen konnen für die orale, parenterale, rektale oder intranasale Verabreichung oder in einer für die Verabreichung durch Inhalation oder Insufflation geeigneten Weise formuliert werden. Zubereitungen der Verbindungen für die orale Verabreichung sind bevorzugt.

Die pharmazeutischen Zubereitungen für die orale Verabreichung können in Form von beispielsweise Tabletten oder Kapseln, die nach an sich bekannten Verfahren mit pharmazeutisch annehmbaren Verdünnungsmitteln, wie Bindemitteln (zum Beispiel vorgelatinisierter Maisstärke, Polyvinylpyrrolidon oder Hydroxypropylmethylcellulose), Füllstoffen (zum Beispiel Lactose, Saccharose, Mannit, Maisstärke, mikrokristalline Cellulose oder Calciumhydrogenphosphat); Schmiermitteln (zum Beispiel Stearinsäure, Polyethylenglykol, Magnesiumstearat, Talk oder Silicidumdioxid); Desintegrationsmitteln (zum Beispiel Kartoffelstärke, Natriumstärkeglykolat oder Natriumcarboxymethylcellulose); oder Benetzungsmitteln (zum Beispiel Natriumlaurylsulfat), hergestellt werden, vorliegen. Die Tabletten können nach an sich bekannten Verfahren überzogen werden. Flüssige Zubereitungen für die orale Verabreichung können in Form von beispielsweise wäßrigen oder öligen Lösungen, Sirupen, Elixieren, Emulsionen oder Suspensionen vorliegen, oder sie können als Trockenprodukt für die Konstitution mit Wasser oder einem anderen geeigneten Träger vor der Verwendung vorliegen. Solche flüssigen Zubereitungen können nach an sich bekannten Verfahren mit pharmazeutisch annehmbaren Zusatzstoffen hergestellt werden, wie Suspensionsmitteln (zum Beispiel Sorbitsirup, Cellulosederivate, Glucose/Zucker-Sirup, Gelatine, Aluminiumstearatgel oder hydrierten genießbaren Fetten); Emulgiermitteln (zum Beispiel Lecithin, Acacia oder Sorbitan-monooleat); nichtwäßrigen Trägern (zum Beispiel Mandelöl, ölige Ester, Ethylalkohol oder fraktionierte Pflanzenöle); und Konservierungsmitteln (zum Beispiel Methyl- oder Propyl-p-hydroxybenzoate oder Sorbinsäure). Die flüssigen Zubereitungen können auch an sich bekannten Puffer, Geschmacks- bzw. Aromamittel, Farbstoffe und Süßstoffe, je nach Bedarf, enthalten.

Für die parenterale Verabreichung können die Verbindungen durch Injektion, bevorzugt intravenöse, intramuskuläre oder subkutane Injektion, formuliert werden. Zubereitungen für die Injektion können in Dosiseinheitsform, zum Beispiel in Ampullen oder Mehrfachdosis-Behältern, mit einem zugegebenen Konservierungsstoff vorliegen. Die Zubereitungen können in Form von Suspensionen, Lösungen oder Emulsionen in öligen oder wäßrigen Trägern vorliegen und Zubereitungshilfsmittel enthalten, wie Suspendier-, Stabilisier- und/oder Dispersionsmittel, und/oder Mittel zur Einstellung der Tonizität der Lösung enthalten. Alternativ kann der aktive Bestandteil in Pulverform für die Konstitution mit einem geeigneten Träger, zum Beispiel sterilem pyrogenfreien Wasser, vor der Verwendung vorliegen.

Die Verbindungen können ebenfalls als rektale Zubereitunggen, wie Suppositorien, formuliert werden, zum Beispiel solche, die an sich bekannte Suppositorien-Grundstoffe, wie Kakaobutter oder andere Glyceride, enthalten.

Für die intranasale Verabreichung können die Verbindungen als flüssige Sprays oder in Form von Tropfen verwendet werden.

Für die Verabreichung durch Inhalation werden die Verbindungen zweckdienlich in Form eines Aerosolsprays aus einer unter Druck stehenden Packung unter Verwendung geeigneter Treibmittel oder in einer Zerstäubungsvorrichtung abgegeben. Im Falle eines unter Druck stehenden Aerosols wird die Dosiseinheit bestimmt, indem ein Ventil vorgesehen wird, welches eine abgemessene Menge freigibt. Kapseln und Patronen aus beispielsweise Gelatine für die Verwendung in einer Inhaltionsvorrichtung oder einer Insufflationsvorrichtung können so zubereitet werden, daß sie ein Pulvergemisch aus einer erfindungsgemäß verwendeten Verbindung und einem geeigneten Pulver-Grundstoff, wie Lactose oder Stärke, enthalten.

Die folgenden Beispiele erläutern die erfindungsgemäße Verwendung.

**Beispiel 1**

Tabletten für die orale Verabreichung

A. Direkte Kompression

(1)

| | |
|---|---|
| Wirkstoff: Curcuminderivat | 15 - 30 mg/Tablette |
| (bzw. pulverisierte Droge | 0,5 - 1,0 g/Tablette) |
| Magnesiumstearat BP | 0,65 mg/Tablette |
| wasserfreie Lactose | 80 mg/Tablette |

Der Wirkstoff wird mit der wasserfreien Lactose und dem Magnesiumstearat vermischt, und das Gemisch wird gesiebt. Das entstehende Gemisch wird zu Tabletten unter Verwendung einer Tablettiermaschine verpreßt.

(2)

| | |
|---|---|
| Wirkstoff: Curcuminderivat | 15 - 30 mg/Tablette |
| (bzw. pulverisierte Droge | 0,5 - 1,0 g/Tablette) |
| Magnesiumstearat BP | 0,7 mg/Tablette |
| mikrokristalline Cellulose NF | 100 mg/Tablette |

Der Wirkstoff wird gesiebt und mit der mikrokristallinen Cellulose und dem Magnesiumstearat vermischt. Das entstehende Gemisch wird unter Verwendung einer Tablettiermaschine zu Tabletten verpreßt.

B. Nasse Granulierung

| | |
|---|---|
| Wirkstoff: Curcuminderivat | 15 - 30 mg/Tablette |
| (bzw. pulverisierte Droge | 0,5 - 1,0 g/Tablette) |
| Lactose BP | 150,0 mg/Tablette |
| Stärke BP | 30,0 mg/Tablette |
| vorgelatinisierte Maisstärke BP | 15,0 mg/Tablette |
| Magnesiumstearat BP | 1,5 mg/Tablette |

Der Wirkstoff wird durch ein geeignetes Sieb gesiebt und mit der Lactose, der Stärke und der vorgelatinisierten Maisstärke vermischt. Geeignete Volumina an gereinigtem Wasser werden zugegeben, und das Pulver wird granuliert. Nach dem Trocknen wird das Granulat gesiebt und mit dem Magnesiumstearat vermischt. Das Granulat wird dann unter Verwendung von Lochstanzen mit geeignetem Durchmesser zu Tabletten verpreßt.

Tabletten anderer Zusammensetzung können hergestellt werden, indem man das Verhältnis von Wirkstoff zu Lactose oder das Kompressionsgewicht ändert und entsprechende Lochstanzen verwendet.

**Beispiel 2**

Kapseln

| | |
|---|---|
| Wirkstoff: Curcuminderivat | 15 - 30 mg/Kapsel |
| (bzw. granulierte Droge | 0,5 - 1,0 g/Kapsel) |
| Stärke 1500 | 150,00 mg/Kapsel |
| Magnesiumstearat BP | 1,00 mg/Kapsel |

Der Wirkstoff wird gesiebt und mit den anderen Bestandteilen vermischt. Das Gemisch wird unter Verwendung einer geeigneten Vorrichtung in Hartgelatinekapseln Nr. 2 gefüllt. Andere Kapseln können hergestellt werden, indem man das Füllgewicht ändert und erforderlichenfalls die Kapselgröße entsprechend ändert.

**Beispiel 3**

Sirup

| Saccharose-freie Zubereitung | | mg/5 ml Dosis |
|---|---|---|
| Wirkstoff: Curcuminderivat | | 15 - 30 |
| Hydroxypropylmethylcellulose USP (Viskositäts-Typ 4000) | | 22,5 |
| Puffer ) | | |
| Geschmacksstoff ) | | |
| Farbstoff ) | | nach Bedarf |
| Konservierungsmittel ) | | |
| Süßstoff ) | | |
| gereinigtes Wasser | auf | 5,0 ml |

Die Hydroxypropylmethylcellulose wird in heißem Wasser dispergiert, abgekühlt und dann mit einer wäßrigen Suspension vermischt, die den Wirkstoff und die anderen Komponenten der Zubereitung enthält. Die entstehende Lösung wird auf ihr Volumen eingestellt und vermischt.

**Beispiel 4**

| Suspension | | mg/5 ml Dosis |
|---|---|---|
| Wirkstoff: Curcuminderivat (bzw. pulverisierte Droge (getrockneter Drogenextrakt entsprechend) | | 15 - 30 0,5 - 1,0 g) |
| Aluminiummonostearat | | 75,00 |
| Süßstoff ) | | |
| Geschmacksstoff ) | | nach Bedarf |
| Farbstoff ) | | |
| fraktioniertes Kokosnußöl | auf | 5,00 |

Das Aluminiummonostearat wird in etwa 90% des fraktionierten Kokosnußöls dispergiert. Die resultierende Suspension wird unter Rühren auf 115 °C erhitzt und dann abgekühlt. Die Süß-, Geschmacks- und Farbstoffe werden zugesetzt, und der Wirkstoff wird dispergiert. Die Suspension wird mit dem restlichen fraktionierten Kokosnußöl auf das Volumen eingestellt und vermischt.

**Beispiel 5**

Sublinguale Tablette

| | |
|---|---|
| Wirkstoff: Curcuminderivat<br>(bzw. Drogenextrakt<br>verpreßbarer Zucker NF<br>Magnesiumstearat BP | 15 - 30 mg/Tablette<br>0,5 - 1,0 g/Tablette)<br>50,5 mg/Tablette<br>0,5 mg/Tablette |

Der Wirkstoff wird durch ein geeignetes Sieb gesiebt, mit den anderen Bestandteilen vermischt und unter Verwendung geeigneter Lochstanzen verpreßt. Tabletten anderer Stärke können hergestellt werden, indem man das Verhältnis von Wirkstoff zu Träger oder das Kompressionsgewicht ändert.

**Beispiel 6**

Suppositorien für die rektale Verabreichung

| | |
|---|---|
| Wirkstoff: Curcuminderivat<br>Witepsol H15+ auf | 15 - 30 mg<br>1,0 g |

+ geeignete Qualität von Adeps solidus Ph.Eur.

Eine Suspension des Wirkstoffs in geschmolzenem Witepsol wird hergestellt und unter Verwendung einer geeigneten Vorrichtung in 1-g-Suppositorienformen eingefüllt.

**Beispiel 7**

Injektion für intravenöse Verabreichung

| | | |
|---|---|---|
| Wirkstoff: Curcuminderivat | | 15 - 30 mg/ml |
| Natriumchlorid-intravenöse Infusion, BP, 0,9% Gew./Vol. | auf | 1 ml |
| Ansatzgröße | 2500 ml | |

Der Wirkstoff wird in einem Teil der Natriumchlorid-intravenösen Infusion gelöst, die Lösung mit der Natriumchloridintravenösen Infusion auf das Volumen eingestellt und die Lösung gründlich vermischt. Die Lösung wird in klare, Typ 1, 10-ml-Glasampullen eingefüllt und unter Stickstoff im Kopfraum durch Abschmelzen des Glases abgesiegelt. Die Ampullen werden durch Erhitzen im Autoklaven bei 120 °C für nicht kürzer als 20 Minuten sterilisiert.

**Beispiel 8**

Patrone für die Inhalation

| | |
|---|---|
| Wirkstoff (mikronisiert): Curcuminderivat<br>Lactose BP | 15 - 30 mg/Patrone<br>25,00 |

Der Wirkstoff wird in einer Energiemühle zu einem feinen Teilchengrößenbereich mikronisiert und dann mit der Lactose vermischt. Die Pulvermischung wird in Hargelatinekapseln Nr. 3 eingefüllt.

**Beispiel 9**

Nasenspray

| | | | |
|---|---|---|---|
| Wirkstoff: Curcumin | | | 1,5 - 3,0 %/Vol. |
| Konservierungsmittel | ) | | nach Bedarf |
| Natriumchlorid BP | ) | | |
| gereinigtes Wasser BP | | auf | 100 |
| Abgabegewicht | | | 100 mg (Äquivalent zu 7 mg Wirkstoff) |

Der Wirkstoff, der Konservierungsstoff und das Natriumchlorid werden in einem Teil des Wassers gelöst. Die Lösung wird mit Wasser auf das Volumen eingestellt und die Lösung gründlich vermischt.

**Patentansprüche**

1. Verwendung von Präparaten der Pflanze Curcuma longa oder von anderen Pflanzen, die Curcumin enthalten, Curcumin oder Curcuminderivaten zur Prophylaxe und/oder Bekämpfung von Krankheiten, die mit einer übermäßigen Bildung von Leukotrienen und/oder Prostaglandinen einhergehen.

2. Verwendung nach Anspruch 1, dadurch **gekennzeichnet,** daß Curcumin oder Curcumin enthaltende Präparate verwendet werden.

3. Verwendung nach Anspruch 1 oder 2, dadurch **gekennzeichnet,** daß die Verwendung bei chronisch entzündlichen Darmerkrankungen erfolgt.

4. Verwendung nach mindestens einem der vorhergehenden Ansprüche, dadurch **gekennzeichnet,** daß die Verwendung bei Colitis ulcerosa oder Morbus Crohn erfolgt.

5. Verwendung nach Anspruch 1 oder 2, dadurch **gekennzeichnet,** daß die Verwendung bei chronischer Hepatitis, chronischem Asthma bronchiale oder Psoriasis erfolgt.

6. Verwendung nach mindestens einem der vorhergehenden Ansprüche, dadurch **gekennzeichnet,** daß die Verwendung intraperitoneal, oral, rektal, intramuskulär, topisch, subkutan, intraartikulär oder intravenös erfolgt.

7. Verwendung nach mindestens einem der vorhergehenden Ansprüche, dadurch **gekennzeichnet,** daß die Verwendung in Form von Tabletten, Dragees, Kapseln, Lösungen, Emulsionen, Salben, Cremes, Inhalaten, Aerosolen oder Suppositorien erfolgt.

8. Verwendung nach mindestens einem der vorhergehenden Ansprüche, dadurch **gekennzeichnet,** daß die Verwendung zusammen mit anderen chemisch reinen Arzneistoffen und/oder anderen pflanzlichen Arzneimitteln erfolgt.

9. Verwendung von Präparaten der Pflanze Curcuma longa oder von anderen Pflanzen, die Curcumin enthalten, Curcumin oder Curcuminderivaten zur Herstellung von Arzneimitteln für die Prophylaxe und/oder Bekämpfung von Krankheiten, die mit einer übermäßigen Bildung von Leukotrienen und/oder Prostaglandinen einhergehen.

10. Verwendung nach Anspruch 9, dadurch **gekennzeichnet,** daß Curcumin und/oder Curcumin enthaltende Präparate verwendet werden.

11. Verwendung nach Anspruch 9 oder 10, dadurch **gekennzeichnet,** daß die Verwendung bei chronisch entzündlichen Darmerkrankungen erfolgt.

12. Verwendung nach mindestens einem der Ansprüche 9 bis 11, dadurch **gekennzeichnet,** daß die Verwendung bei Colitis ulcerosa oder Morbus Crohn erfolgt.

13. Verwendung nach Anspruch 11 oder 12, dadurch **gekennzeichnet,** daß die Verwendung bei chronischer Hepatitis, chronischem Asthma bronchiale oder Psoriasis erfolgt.

14. Verwendung nach mindestens einem der Ansprüche 9 bis 13, dadurch **gekennzeichnet,** daß die Verwendung intraperitoneal, oral, rektal, intramuskulär, topisch, subkutan, intraartikulär oder intravenös erfolgt.

15. Verwendung nach mindestens einem der Ansprüche 9 bis 14, dadurch **gekennzeichnet,** daß die Verwendung in Form von Tabletten, Dragees, Kapseln, Lösungen, Emulsionen, Salben, Cremes, Inhalaten, Aerosolen oder Suppositorien erfolgt.

16. Verwendung nach mindestens einem der Ansprüche 9 bis 15, dadurch **gekennzeichnet,** daß die Verwendung zusammen mit anderen chemisch reinen Arzneistoffen und/oder anderen pflanzlichen Arzneimitteln erfolgt.

# Fig. 1

KOZENTRATIONSABHÄNGIGE HEMMUNG DER 5 - LIPOXYGENASE DURCH CURCUMIN IN CALCIUM / IONOPHORE - STIMULIERTEN INTAKTEN PERITONEALEN NEUTRO - PHILEN GRANULOCYTEN DER RATTE DURCH CURCUMIN (MITTELWERTE ± SD)

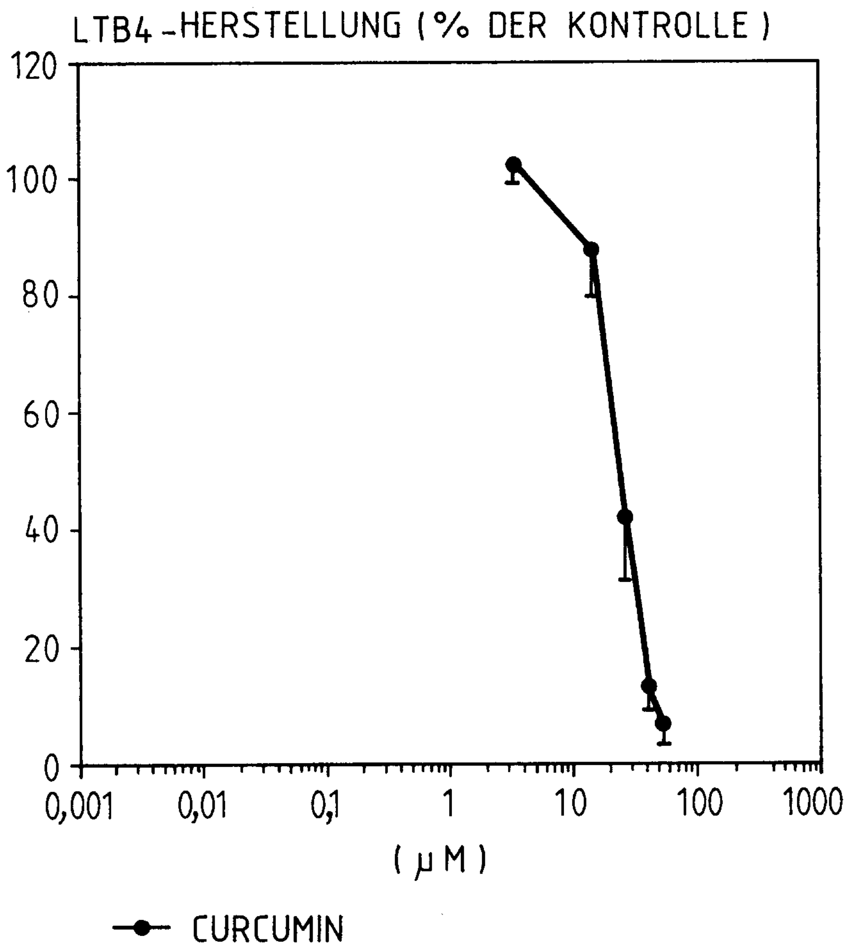

# Fig. 2

KOZENTRATIONSABHANGIGE HEMMUNG DER CYCLOOXYGENASE (□) UND DER 12-LIPOXYGENASE (◇) DURCH CURCUMIN IN DER CALCIUM/IONOPHORE-STIMULIERTEN INTAKTEN HUMANEN BLUTPLÄTTCHEN (MITTELWERTE ± SD)

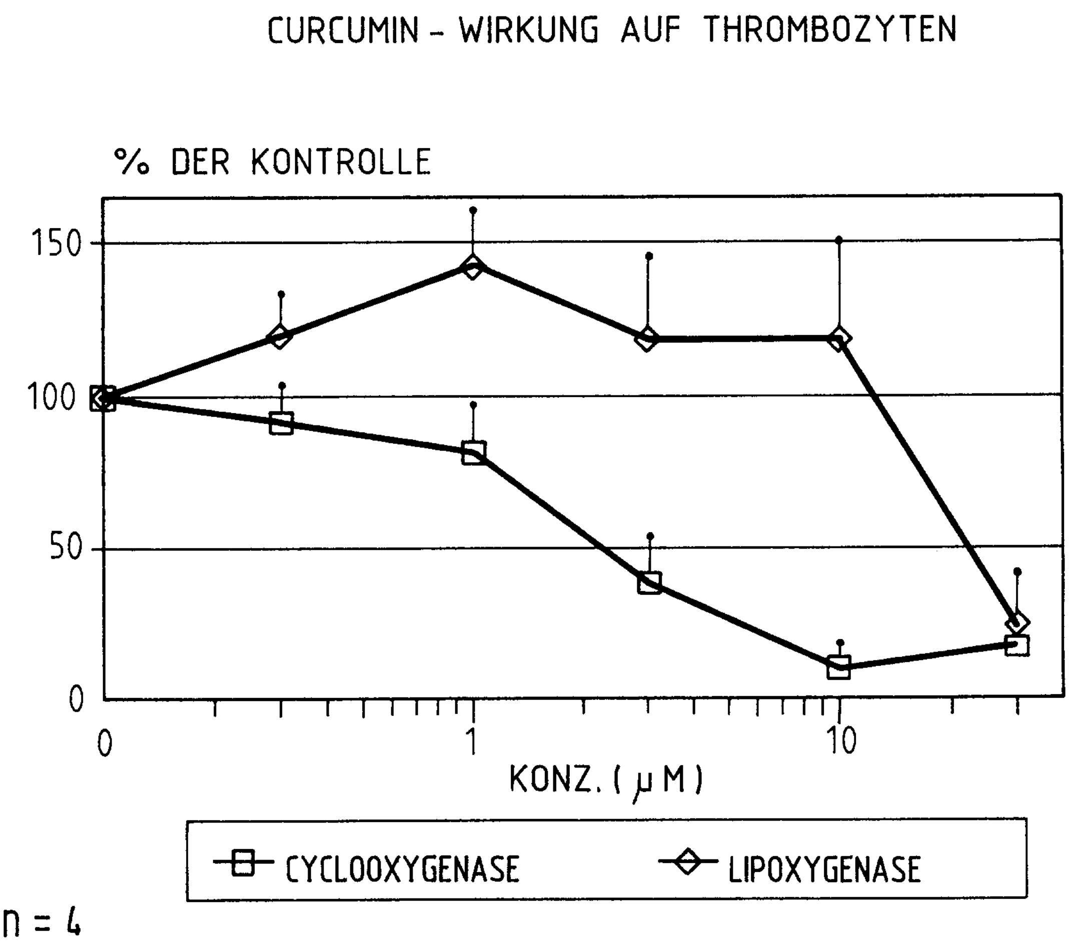


n = 4

# Fig. 3

KONZENTRATIONSABHÄNGIGE HEMMUNG DER DURCH PEROXIDATION DER ARACHIDONSÄURE MITTELS EISEN / ASCORBAT ENTSTEHENDEN PRODUKTE DURCH CURCUMIN ( MITTELWERT±SD )

ANTIOXIDATIVE WIRKUNG VON CURCUMIN

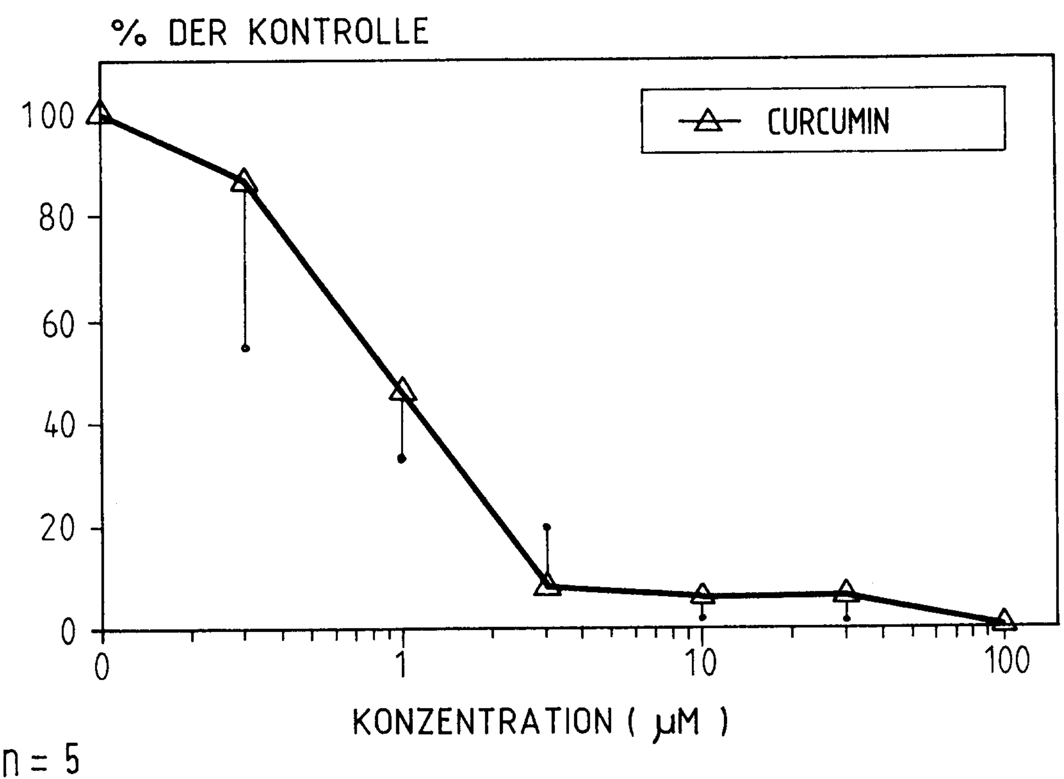


n = 5